# EUROPÄISCHE PATENTSCHRIFT

(11) **EP 0 638 062 B1**
(45) Veröffentlichungstag und Bekanntmachung des Hinweises auf die Patenterteilung: **08.05.1996**
(21) Anmeldenummer: 90915116.9
(22) Anmeldetag: 13.10.1990
(51) Int. Cl.: C07C 69/675, C07C 67/31, C07C 67/26

(54) **VERFAHREN ZUR HERSTELLUNG VON ESTERPOLYOLE ENTHALTENDEN REAKTIONSMISCHUNGEN**
PROCESS FOR MANUFACTURING REACTION MIXTURES CONTAINING ESTER POLYOLS
PROCEDE DE FABRICATION DE MELANGES REACTIONNELS RENFERMANT DES ESTERPOLYOLS

(30) Priorität: 21.10.1989 DE 3935127
(43) Veröffentlichungstag der Anmeldung: 15.02.1995
(73) Patentinhaber: Henkel Kommanditgesellschaft auf Aktien, D-40191 Düsseldorf (DE)
(72) Erfinder: STOLL, Gerhard, D-4052 Korschenbroich 1 (DE); DAUTE, Peter, D-4300 Essen 1 (DE); HÖFER, Rainer, D-4000 Düsseldorf 30 (DE); GRÜTZMACHER, Roland, D-5603 Wülfrath (DE); KLUTH, Hermann, D-4000 Düsseldorf 20 (DE)
(86) Internationale Anmeldenummer: EP9001737
(87) Internationale Veröffentlichungsnummer: WO9105759

(56) Entgegenhaltungen:
- BE-A- 681 520
- US-A- 2 909 537

## Beschreibung

Gegenstand der vorliegenden Erfindung ist ein Verfahren zur Herstellung von vergleichsweise niedrigviskosen Esterpolyolen enthaltenden Reaktionsmischungen durch Umsetzung von epoxidierten Estern und/oder Alkoholen mit Carbonsäuren.

Ein weiteres Ziel der Erfindung sind die nach obigem Verfahren herstellbaren Mischungen von Esterpolyolen, die zum überwiegenden Anteil Esterpolyole enthalten, die freie Hydroxylgruppen in Nachbarstellung zur Estergruppe aufweisen.

Polyole sind wichtige und vielfältige Rohstoffe mit breiten Anwendungsmöglichkeiten.

Ein kostengünstiges Verfahren zur Herstellung von Polyolen ist die ringöffnende Umsetzung von mehrfach epoxidierten Verbindungen mit protischen Reaktanden.

Die Ringöffnung von epoxidierten Ölen oder Fetten mit Alkoholen als Protonendonatoren wird beispielsweise in der DE-PS 29 00 030, in der EP-PS 113 798 und in den US-PS 3 475 499 und 3 607 778 beschrieben.

In der DE-OS 33 18 596 wird in einer Ausführungsform die Ringöffnung von epoxidierten Carbonsäureestern mit mehrwertigen Alkoholen beschrieben. Zur Unterdrückung von Mehrfachumsetzungsprodukten, die durch Brückenbildung der freien Hydroxylgruppen des Alkoxyrestes der Hydroxylalkoxycarbonsäuren mit 2 oder mehr Fettsäure-Molekülen entstehen, werden gezielte Reaktionsbedingungen gewählt. So wird das epoxidierte Fettsäurederivat zu einem hohen Überschuß an mehrfunktionellen Alkoholen, bevorzugt in einem Überschuß von 0,5 mol bis zu mehr als 10 mol Alkohol/mol epoxidierten Fettsäurederivats, eindosiert.

Zur Beschleunigung der Ringöffnung von epoxidierten Verbindungen mit Alkoholen werden gewöhnlich Säuren als Katalysatoren zugegeben, die jedoch nach der Reaktion abgetrennt werden müssen oder nach Neutralisation mit Basen im Polyol verbleiben, und bei dessen Verwendung in Polyurethanmassen zu Austrübungen und Schrumpfungen führen können.

Ohne Katalysatoren kann die Ringöffnung von epoxidierten Ölen oder Fetten direkt mit Carbonsäuren erreicht werden, wobei Hydroxy- und Acyloxygruppen modifizierte Reaktionsmischungen entstehen.

Die Ringöffnung von Epoxidgruppen enthaltendem Sojaöl mit Acrylsäure bzw. Methacrylsäure wird beispielsweise in der US-PS 4 016 059 beschrieben. Die Umsetzung erfolgt nach dem sogenannten Eintopfverfahren, wonach die gesamte Menge aller Reaktanden gleichzeitig zur Reaktion gebracht werden, und führt zu einem Hydroxy-(meth)acryloxysojaölester enthaltenden Reaktionsgemisch. Die genaue Zusammensetzung des Reaktionsgemisches wird u. a. durch die eingesetzte Menge an Acrylsäure bedingt. Bei Zugabe von Acrylsäure im Unterschuß - bezogen auf Epoxidgehalt im Sojaöl - können Restgehalte an Epoxidgruppen bis zu 5,2 Gew.-% auftreten. Beim Arbeiten mit einem Überschuß an Acrylsäure kann zwar der Restgehalt an Epoxid bis 0,2 Gew.-% reduziert werden, aber die Umsetzungen werden stets in Anwesenheit eines Lösungsmittels wie Ethylbenzol durchgeführt.

Vergleichbare Umsetzungen von epoxidiertem Sojaöl und Acrylsäure u. a. im molaren Verhältnis 1:3 werden in der US-PS 3 125 592 beschrieben. Dort werden nach dem Eintopfverfahren ohne Verwendung von Lösungsmitteln Hydroxyacryloxysojaölester enthaltende Reaktionsmischungen als viskose Flüssigkeiten erhalten.

Ebenfalls viskose Flüssigkeiten werden bei der Umsetzung von epoxidiertem Sojaöl mit Leinölfettsäure oder dehydratisierter Ricinolsäure nach dem Eintopfverfahren erhalten (US-PS 2 909 537). Anscheinend unabhängig von der verwendeten Fettsäure und ihren eingesetzten Mengen entstehen Gemische mit nicht näher offenbarten Viskositäten. Die erhaltenen Gemische weisen jedoch noch unumgesetzte Leinölfettsäure oder dehydratisierte Ricinolsäure auf, die z. B. bei ihrer Weiterverwendung in Polyurethanmassen stören.

Eine anteilige Ringöffnung von epoxidierten Fetten, die mehr als eine Epoxidgruppe im Molekül enthalten, mit Di-, Polycarbonsäuren oder deren Anhydriden in nahezu stöchiometrischen Mengen wird in der US-PS 33 180 749 beschrieben. Nach dem dort beanspruchten Herstellungsverfahren soll die nach dem Eintopfverfahren durchgeführte Reaktion vor Verbrauch der gesamtem Carbonsäuremenge abgebrochen werden, damit zumindest noch eine unumgesetzte Epoxidgruppe in jedem Fettmolekül verbleibt.

Die nach dem Eintopfverfahren erhaltenen Ringöffnungsgemische von epoxidiertem Sojabohnenöl mit Säuren sind meist vergleichsweise hochviskose Produkte, die daher vor ihrer Verwendung z. B. als Korrosionsschutzmittel (US-PS 2 909 537) oder in Polyurethanmassen (US-PS 4 016 059) mit Lösungsmitteln verdünnt oder erwärmt werden mußten.

Aufgabe der Erfindung ist es, vergleichsweise niedrigviskose Esterpolyole enthaltende Reaktionsmischungen durch Umsetzung von epoxidierten Verbindungen mit Carbonsäuren herzustellen.

Überraschenderweise wurde gefunden, daß Esterpolyole enthaltende Reaktionsgemische mit vergleichsweise niedrigen Viskositäten hergestellt werden können, wenn die Carbonsäuren in wenigstens stöchiometrischen Mengen vorgelegt und die epoxidierten Ester oder Alkohole zeitverzögert zugefügt werden.

Gegenstand der vorliegenden Erfindung ist somit ein Verfahren zur Herstellung von Esterpolyole enthaltenden Reaktionsmischungen durch ringöffnende Umsetzungen von epoxidierten Estern und/oder Alkoholen mit Carbonsäuren, dadurch gekennzeichnet, daß man zur Herstellung vergleichsweise niedrigviskoser Umsetzungsprodukte die Carbonsäuren in wenigstens etwa stöchiometrischen Mengen zum Epoxidgehalt des/der Reaktanden vorlegt, und die epoxidierte Reaktionskomponente derart zeitverzögert eindosiert, daß substantielle Anteile nicht abreagierter Epoxidgruppen im Reaktionsgemisch vermieden werden.

Ein weiteres Ziel der Erfindung sind die nach obigem Verfahren herstellbaren Mischungen von Esterpolyolen, die zum überwiegenden Anteil Esterpolyole enthalten, die freie Hydroxylgruppen in Nachbarstellung zur Estergruppe aufweisen.

Unter den in Rede stehenden Esterpolyolen werden Verbindungen verstanden, die im statistischen Mittel pro Molekül über mehr als eine freie Hydroxylgruppe verfügen und die zumindest anteilsweise in Nachbarstellung zur Hydroxylgruppe eine Estergruppe aufweisen.

Bei der Herstellung der vergleichweise niedrigviskosen Esterpolyole enthaltenden Reaktionsmischungen werden die Carbonsäuren vorgelegt und auf Temperaturen üblicherweise über 80°C und unter 300 °C , bevorzugt über 100 °C und unter 270 °C erwärmt. Die Wahl der Reaktionstemperatur wird u. a. durch die vorgelegten Carbonsäuren bestimmt. So werden bei niederen Carbonsäuren Reaktionstemperaturen des unteren Temperaturbereichs, bei höheren Carbonsäuren die des höheren Temperaturbereichs bevorzugt. Reaktionstemperaturen über 300 °C führen vor allem bei längeren Reaktionszeiten zu einem erhöhten Anteil an kondensierten Produkten und/oder zur Zersetzung des Reaktionsgemisches.

Zu den vorgelegten, erwärmten Carbonsäuren werden die epoxidierten Ester und/oder Alkohole vorzugsweise unter intensivem Rühren eindosiert. Das Eindosieren der epoxidierten Reaktionskomponente erfolgt erfindungsgemäß derart zeitverzögert, daß substantielle Anteile an nicht abreagierten Epoxidgruppen im Reaktionsgemisch vermieden werden. Die Gehalte an nicht abreagierten Epoxidgruppen sollten unter 50 Gew.-% gehalten werden. Der angegebene Wert von unter 50 Gew.-% ist ein relativer Gehaltswert an Epoxidsauerstoff im Reaktionsgemisch nach vollständiger Zugabe der epoxidierten Reaktanden und bezieht sich auf den theoretisch zu erwartenden Wert an Epoxidsauerstoff im Reaktionsgemisch nach dem Eintopfverfahren, wobei die Werte bereinigt sind von der Verdünnung durch vorgelegte Carbonsäure. Deutlich niedrigere Gehalte als 50 Gew.-% kann man erhalten, wenn man die Dosiergeschwindigkeit sehr klein hält. Aus ökonomischen Gründen ist jedoch eine möglichst schnelle Dosierung erwünscht, die vorzugsweise 1,5 Stunden nicht überschreiten sollte.
Bei höheren Gehalten an nichtabreagierten Epoxidgruppen wächst die Wahrscheinlichkeit, daß bereits vorliegende Hydroxylgruppen enthaltende Esterpolyole noch nichtabreagierte Epoxidgruppe über die Hydroxylgruppe öffnen und höher kondensierte Einheiten entstehen. Über Titrationen läßt sich der Gehalt an nicht abreagierten Epoxidgruppen bestimmen, so z.B. nach der Methode von R.R.Jay (Analyt.Chemie 36(1964),667/8).

In einer bevorzugten Ausführungsform erfolgt die zeitverzögerte Dosierung durch Zutropfen der epoxidierten Verbindung. Möglich sind aber auch alle anderen kontrollierbaren Zugabepraktiken.

Nach beendeter Zudosierung der epoxidierten Reaktionskomponente läßt man erfindungsgemäß die Reaktionsmischung bei den obengenannten Reaktionstemperaturen abreagieren, bis ein absoluter Restepoxidsauerstoffgehalt im Reaktionsgemisch, vorzugsweise unter 1,0 Gew.-%, bevorzugt unter 0,5 % und insbesondere unter 0,3 Gew.-% auftritt.

In der Regel wird nach der Abreaktion die ggf. überschüssige Carbonsäure aus dem Reaktionsgemisch entfernt. Bevorzugt wird die destillative Abtrennung der überschüssigen Carbonsäure unter Vakuum, aber auch andere Abtrennungsarten wie Neutralisation mit einer Base und ggf. anschließender Filtration sind möglich. Das erfindungsgemäße Abdestillieren der Carbonsäuren erfordert je nach eingesetzter Carbonsäure und angelegtem Vakuum unterschiedliche Temperaturen, die aber die bevorzugten Reaktionstemperaturen bis 300 °C nicht überschreiten sollten.

In der Regel werden zur Herstellung von Esterpolyole enthaltenden Reaktionsmischungen als epoxidierte Reaktionskomponente epoxidierte Ester und/oder epoxidierte Alkohole eingesetzt, die im statistischen Mittel pro Molekül mehr als eine Epoxidgruppe und vorzugsweise zwei und mehr Epoxidgruppen im Molekül enthalten.

Die Herstellung der epoxidierten Ester und/oder Alkohole erfolgt nach den bekannten Verfahren wie z. B. nach EP 286 937 oder der DE-PS 1 042 565 vollständig oder nahezu vollständig. Im Sinne der Erfindung kann die Epoxidierung mehrfach ungesättigter Ester und/oder Alkohole auch nur teilweise erfolgen, wobei gegeben sein muß, daß die dabei entstehenden epoxidierten Ester und/oder epoxidierten Alkohole im statistischen Mittel pro Molekül mehr als eine Epoxidgruppe enthalten.

Als epoxidierte Ester können Ester von epoxiderten Säuren mit Alkoholen und/oder von epoxidierten Alkoholen mit Säuren eingesetzt werden. Bevorzugt werden epoxidierte Ester von 1- bis 4-wertigen Alkoholen mit bis zu 40 C-Atomen, bevorzugt mit bis zu 36 C-Atomen und insbesondere mit 1 bis 22 C-Atomen wie Methanol, 2-Ethylhexanol, Ethylenglykol, Butandiol, Neopentylglykol, Glycerin und/ oder Pentaerythrit.

Die eingesetzten epoxidierten Ester enthalten vorzugsweise epoxidierte Säuren mit bis zu 40 C-Atomen, bevorzugt mit bis zu 36 C-Atomen und insbesondere mit bis zu 22 C-Atomen. Als Ausgangsstoffe für epoxiderte Ester eignen sich die zahlreichen tierischen und/oder pflanzlichen Triglyceride wie Rindertalg, Palmöl, Schmalz, Erdnußöl, Rüböl, Baumwollsaatöl, Sojabohnenöl, Tranöl, Sonnenblumenöl, Korianderöl und/oder Leinöl. Besonders bevorzugte epoxiderte Ester sind epoxidiertes Sojabohnenöl (Epoxidsauerstoffgehalt 5,8 - 6,5 Gew.-%), ölsäurereiches und/oder ölsaurearmes, epoxidiertes Sonnenblumenöl (Epoxidsauerstoffgehalt 4,4 - 6,6 Gew.-%), epoxidiertes Leinöl (Epoxidsauerstoffgehalt 8,2 - 8,6 Gew.-%) sowie epoxidiertes Tranöl (Epoxidsauerstoffgehalt 6,3-6,7 Gew.-%).

Als epoxidierte Alkohole werden vorzugsweise solche mit 3 bis 32 C-Atomen, bevorzugt mit über 12 C-Atomen und insbesondere bis 22 C-Atomen eingesetzt. Besonders bevorzugt werden solche epoxidierten Alkohole, die mehr als eine, vorzugsweise 2 und/oder 3 Epoxidgruppen pro Molekül enthalten und/oder Gemische von epoxidierten Alkoholen, die im statistischen Mittel über mehr als eine Epoxidgruppe pro Molekül verfügen. Unter den genannten Bedingungen können beispielsweise die Epoxyderivate der Alkohole 10-Undecen-1-ol, 9c-Octadecen-1-ol (Oleylalkohol), 9t-Octadecen-1-ol (Elaidylalkohol), 9c-0ctadecen-1.12-diol (Ricinolalkohol), 9c.12c.-Octadecadien-1-ol (Linoleyalkohol), 9c.12c.15c.-0ctadecatrien-1-ol (Linolenylalkohol), 9c-Eicosen-1-ol (Gadoleylalkohol), 13c-Docosen-1-ol (Erucaalkohol) und/oder 13t-Docosen-1-ol (Brassidylalkohol) eingesetzt werden.
Erfindungsgemäß können auch Mischungen von epoxidierten Alkoholen und epoxidierten Estern in jeder beliebigen Abmischung als epoxidierte Reaktionskomponente eingesetzt werden.

Die genannten epoxidierten Reaktionskomponenten werden nach dem erfindungsgemäßen Verfahren mit Carbonsäuren vollständig oder zumindest nahezu vollständig ringgeöffnet. Dazu eignen sich vorzugsweise einbasige Carbonsäuren. Als Carbonsäuren können synthetische, natürliche, aliphatische, aromatische, verzweigte und/oder unverzweigte Carbonsäuren mit bis zu 40 C-Atomen, bevorzugt bis zu 36 C-Atomen und insbesondere mit bis zu 22 C-Atomen eingesetzt werden. Zur Ringöffnung eignen sich gesättigte und ungesättigte Carbonsäuren sowie Mischungen derselben. Besonders bevorzugt werden als Carbonsäure Ameisen-, Essig-, Propion-, Capryl-, Caprin-, Behen-, Palmitolein-, Öl-, Linol-und/oder Linolensäuren vorgelegt. Höhere Anteile an ungesättigten Carbonsäuren ergeben besonders niedrigviskose Esterpolyole enthaltende Reaktionsmischungen.

Die epoxidierten Reaktionskomponenten werden erfindungsgemäß in Mengen bis 1:10, bevorzugt in höchstens äquimolaren Mengen und insbesondere in geringem Unterschuß - berechnet als mol-% Epoxid und bezogen auf mol-% Säuregruppe - zu vorgelegten Carbonsäuren zeitverzögert eindosiert.

In der erfindungsgemäß bevorzugten Ausführungsform werden vorzugsweise über 1 und unter 1,5 Säuremoleküle und bevorzugt über 1 und unter 1,1 Säuremoleküle - bezogen auf eine Epoxidgruppe des epoxidierten Reaktanden - vorgelegt.

Die nach dem erfindungsgemäßen Verfahren hergestellten Esterpolyole enthaltenden Reaktionsgemische sind hellgelbe bis hellbraune Flüssigkeiten mit vergleichsweise niedrigen Viskositäten.

Vorzugsweise haben die erfindungsgemäß hergestellten Reaktionsmischungen bis zu 50 % und bevorzugt zwischen 10 und 40 % - gemessen in mPas nach Höppler (DIN 53 015) bei 20 °C - niedere Viskositätswerte als die nach dem Eintopfverfahren hergestellten.

Die absoluten Viskositätswerte der erfindungsgemäß hergestellten Reaktionsmischungen sind stark abhängig von dem Molekulargewicht und der chemischen Konstitution der epoxidierten Reaktanden und der vorgelegten Carbonsäuren. Die in einer besonderen Ausführungsform durch Ringöffnung von epoxidierten Fetten und/oder Ölen mit Ölsäure hergestellten Reaktionsmischungen weisen Viskositäten deutlich unter 2 000 mPas, insbesondere unter 1 500 mPas auf. Umsetzungsprodukte von epoxidierten Fetten und/oder Ölen mit ungesättigten Fettsäuren wie Linol-, Linolensäure oder Fettsäuregemischen wie Leinölfettsäure dürften vergleichbare Viskositäten aufweisen.

Die nach dem erfindungsgemäßen Verfahren hergestellten Esterpolyolmischungen enthalten im statistischen Mittel mehr als eine, bevorzugt mehr als 1,5 freie Hydroxylgruppen pro Molekül. In Nachbarstellung zur Hydroxylgruppe weisen die Mischungen der Esterpolyole Estergruppen auf, wenn die Ringöffnung der Epoxide durch die vorgelegte Carbonsäure erfolgte. Der Rest der Mischung enthält durch Epoxidringöffnung mit bereits vorliegenden Esterpolyolen dimere, trimere und/oder höhere Kondensate. Nach der Gelpermeationschromatographie (GPC) (Standard (Poly-)Styrol) kann man erkennen, daß die nach dem Eintopfverfahren hergestellten Esterpolyolmischungen einen größeren Anteil an höheren Kondensaten zeigen.

Das Verfahren erlaubt es, Produkte herzustellen, die zum überwiegenden Anteil nichtkondensierte Esterpolyole enthalten, die im statistischen Mittel vorzugsweise mehr als eine freie Hydroxylgruppe und benachbart zu dieser eine Estergruppe aufweisen.

Die nach dem erfindungsgemäßen Verfahren herstellbaren Mischung enthalten monomere Esterpolyole mit zur freien Hydroxylgruppe benachbarten Estergruppen von synthetischen, natürlichen, aliphatischen, aromatischen, verzweigten und/oder unverzweigten Carbonsäuren mit bis zu 40 C-Atomen, insbesondere solche des Bereichs von 8 C-Atomen bis 22 C-Atomen. Gesättigte und ungesättigte Carbonsäuren werden gleichermaßen bevorzugt, insbesondere Capryl-, Caprin-, Öl-, Linol- und/oder Linolensäure.

In den folgenden Beispielen wird die erfindungsgemäße Herstellung der Esterpolyole enthaltenden Reaktionsmischungen beschrieben. Die Kenndaten der hergestellten Esterpolyolmischungen wie Hydroxylzahl, Säurezahl, Restepoxidgehalt, Viskosität nach Höppler bei 20 °C usw. werden in Tabelle 1 zusammengefaßt und den in dem nach dem Eintopfverfahren hergestellten Esterpolyolmischungen vergleichend gegenübergestellt.

### Beispiel 1

823 g technische Ölsäure (SZ = 200,5, JZ = 94), die bezogen auf Säurezahl 2,9 mol ensprechen, wurden im Reaktionsgefäß unter Rühren auf 160 °C erwärmt. Unter Rühren wurden dazu 661 g Sojaölepoxid (Epoxidsauerstoffgehalt 6,78 Gew.-%), entsprechend 2,8 mol bezogen auf Epoxidgehalt, so zeitverzögert zudosiert (75 Minuten), daß der absolute Gehalt an nicht abreagierten Epoxidgruppen im Reaktionsgemisch nicht 1,44 Gew.-% überstieg. Nach erfolgter Zudosierung wurde das Reaktionsgemisch solange (3 Stunden) bei 160°C gehalten, bis der Restepoxidsauerstoffgehalt unter 0,3 Gew.-% sank. Die unumgesetzte Ölsäure (96 g). wurde im Vakuum (kleiner 10 Pas) bis 200 °C abdestilliert. Man erhielt das Polyolgemisch als klare, dunkelgelbe Flüssigkeit mit den Kennzahlen : OHZ = 40,7; VZ = 194; JZ = 54,7 ; SZ = 6,5.

### Beispiel 2

1225 g Vorlauffettsäure (60 % C₈, 35 % C₁₀, SZ = 361,9), die bezogen auf Säurezahl 7,9 mol entsprechen, wurden im Reaktionsgefäß vorgelegt und unter Rühren auf 150 °C erwärmt. Unter Rühren wurden 1770 g Sojaölepoxid (Epoxidsauerstoffgehalt 6,78 Gew.-%), entsprechend 7,5 mol bezogen auf Epoxidgehalt, so zeitverzögert zudosiert (60 Minuten), daß der absolute Gehalt an nicht abreagierten Epoxidgruppen im Reaktionsgemisch nicht 1,6 Gew.-% überstieg. Nach erfolgter Zudosierung wurde die Reaktionstemperatur langsam auf 170 °C erhöht und das Reaktionsgemisch solange (2 Stunden) bei dieser Temperatur gehalten, bis der Restepoxidsauerstoffgehalt unter 0,15 Gew.-% sank. Die unumgesetzte Vorlauffettsäure (470 g) wurde im Vakuum (kleiner 10 Pas) bis 200 °C abdestilliert, Man erhielt das Polyolgemisch als klare, gelbe Flüssigkeit mit den Kennzahlen : OHZ = 96; VZ = 235; SZ = 1,5.

### Beispiel 3

948 g technische Monomerfettsäure - ein Produktgemisch erhalten nach M.J.A.M. den Otter (Fette, Seifen, Anstrichmittel 8 /1970, 667-673) (SZ = 174, JZ = 70,1), die bezogen auf Säurezahl 3,9 mol entsprechen, wurden im Reaktionsgefäß unter Rühren auf 160 °C erwärmt. Unter Rühren wurden dazu 661 g Sojaölepoxid (Epoxidsauerstoffgeahlt 6,78 Gew.-%), entsprechend 2,8 mol bezogen auf Epoxidgehalt, so zeitverzögert eindosiert (60 Minuten), daß der absolute Gehalt an nicht abreagierten Epoxidgruppen im Reaktionsgemisch nicht 1,33 Gew.-% überstieg. Nach erfolgter Eindosierung wurde das Reaktionsgemisch solange (3 Stunden) bei 160 °C gehalten, bis der Restepoxidsauerstoffgehalt unter 0,28 Gew.-% sank. Die unumgesetzte technische Monomerfettsäure (164 g) wurde im Vakuum (kleiner 10 Pas) bis 270 °C abdestilliert. Man erhielt das Esterpolyolgemisch als klare, dunkelgelbe Flüssigkeit mit den Kennzahlen : OHZ = 40,1; VZ = 190,9; JZ = 37,9; SZ = 8,5.

### Beispiel 4

234 g Propionsäure, die bezogen auf die Säurezahl 3,15 mol entsprechen, wurden im Reaktionsgefäß unter Rühren auf 140 °C erwärmt. Unter Rühren wurden dazu 713 g Sojaölepoxid (Epoxidsauerstoffgehalt 6,73 Gew.-%), entsprechend 3,0 mol bezogen auf Epoxidgehalt, so zeitverzögert eindosiert (83 Minuten), daß der absolute Gehalt an nicht abreagierten Epoxidgruppen im Reaktionsgemisch nicht 1,67 Gew.- % überstieg. Nach erfolgter Eindosierung wurde das Reaktionsgemisch solange (5 Stunden) bei 140 °C gehalten, bis der Restepoxidsauerstoffgehalt unter 0,18 Gew.-% sank. Die unumgesetzte Propionsäure (114 g) wurde im Vakuum (kleiner 10 Pas) bis 190 °C abdestilliert. Man erhielt das Esterpolyolgemisch als klare, dunkelgelbe Flüssigkeit mit den Kennzahlen: OHZ = 129; VZ = 277,5; JZ = 4,3; SZ = 2,1.

### Beispiel 5

631 g Vorlauffettsäure (60 % C₈, 35 % C₁₀, SZ = 358) wurden im Reaktionsgefäß vorgelegt und unter Rühren auf 150°C erwärmt. Unter Rühren wurden 1043 g eurucasäurearmes Rübölepoxid (Epoxidsauerstoffgehalt 5,95 Gew.-%) so zeitverzögert zudosiert (60 Minuten), daß der absolute Gehalt an nichtabreagierten Epoxidgruppen im Reaktionsgemisch nicht 1,6 Gew.-% überstieg. Nach erfolgter Zudosierung wurde die Reaktionstemperatur langsam auf 170°C erhöht und das Reaktionsgemisch so lange (3 Stunden) bei dieser Temperatur gehalten, bis der Restepoxidsauerstoffgehalt unter 0,17 Gew.-% sank. Die unumgesetzte Vorlauffettsäure (201 g) wurde im Vakuum (kleiner 10 Pas) bis 200°C abdestilliert. Man erhielt das Polyolgemisch als klare gelbe Flüssigkeit mit den Kennzahlen: OHZ = 119; VZ = 237; SZ = 3,1 mit einer Viskosität von 6238 mPas (Höppler, 20°C)

### Beispiel 6

490 g Vorlauffettsäure (60 % C₈, 35 % C₁₀, SZ = 361,9) wurden im Reaktionsgefäß vorgelegt und unter Rühren auf 160°C erwärmt. Unter Rühren wurden 716 g Sojaölepoxid (Epoxidsauerstoffgehalt 6,78 Gew.-%) so zeitverzögert zudosiert (6,5 Stunden), daß der absolute Gehalt an nichtabreagierten Epoxidgruppen im Reaktionsgemisch nicht 1,6 Gew.-% überstieg. Nach erfolgter Zudosierung wurde die Reaktionstemperatur langsam auf 170°C erhöht und das Reaktionsgemisch so lange (1 Stunde) bei dieser Temperatur gehalten, bis der Restepoxidsauerstoffgehalt unter 0,17 Gew.-% sank. Die unumgesetzte Vorlauffettsäure (71 g) wurde im Vakuum (kleiner 10 Pas) bis 200°C abdestilliert. Man erhielt das Polyolgemisch als klare gelbe Flüssigkeit mit den Kennzahlen: OHZ = 99; JZ = 3,7; VZ = 239; SZ = 3,9 und mit einer Viskosität von 4160 mPas (Höppler, 20°C).

### Veraleichsbeispiel 1

819 g technische Ölsäure (SZ = 201,5), die bezogen auf die Säurezahl 2,9 mol entsprechen, wurden zusammen mit 666 g Sojaölepoxid (Epoxidsauerstoffgehalt 6,73 Gew.-%), entsprechend 2,8 mol bezogen auf Epoxidgehalt, unter Rühren auf 160 °C erwärmt und solange bei dieser Temperatur gehalten (4 Stunden), bis der Restepoxidsauerstoffgehalt unter 0,18 Gew.-% sank. Die unumgesetzte Ölsäure wurde im Vakuum (kleiner 10 Pas) bis 260 °C abdestilliert. Man erhielt das Esterpolyolgemisch als klare, dunkelgelbe Flüssigkeit mit den Kennzahlen : OHZ = 51; VZ = 192; JZ = 50,4; SZ = 2,3.

### Veraleichsbeispiel 2

234 g Propionsäure, die bezogen auf die Säurezahl 3,15 mol entsprechen, wurden zusammen mit 713 g Sojaölepoxid (Epoxidsauerstoffgehalt 6,73 Gew.-%), entsprechend 3,0 mol bezogen auf Epoxidgehalt, unter Rühren auf 140 °C erwärmt und solange bei dieser Temperatur gehalten (5 Stunden), bis der Restepoxidsauerstoffgehalt unter 0,21 Gew.-% sank. Die unumgesetzte Propionsäure (115 g) wurde im Vakuum (kleiner 10 Pas) bis 185 °C abdestilliert. Man erhielt das Esterpolyolgemisch als klare, dunkelgelbe Flüssigkeit mit den Kennzahlen : OHZ = 126; VZ = 273,8; JZ = 1,8; SZ = 1,8.

### Veraleichsbeispiel 3

1234 g Vorlauffettsäure (60 % C₈ , 35 % C₁₀, SZ = 358), die bezogen auf die Säurezahl 7,9 mol entsprechen, wurden zusammen mit 1762 g Sojaölepoxid (Epoxidsauerstoffgehalt 6,81 Gew.-%), entsprechend 7,5 mol bezogen auf Epoxidgehalt, unter Rühren auf 140 °C erwärmt und langsam auf 170 °C gesteigert. Bei dieser Temperatur wurde die Reaktionsmischung solange gehalten (3 Stunden), bis der Restepoxidsauerstoffgehalt unter 0,12 Gew.-% sank. Die unumgesetzte Vorlauffettsäure (453 g) wurde im Vakuum (kleiner 10 Pas) bis 185 °C abdestilliert. Man erhielt das Esterpolyolgemisch als klare, dunkelgelbe Flüssigkeit mit den Kennzahlen : OHZ = 102,7; JZ = 3,6; SZ = 5,0.

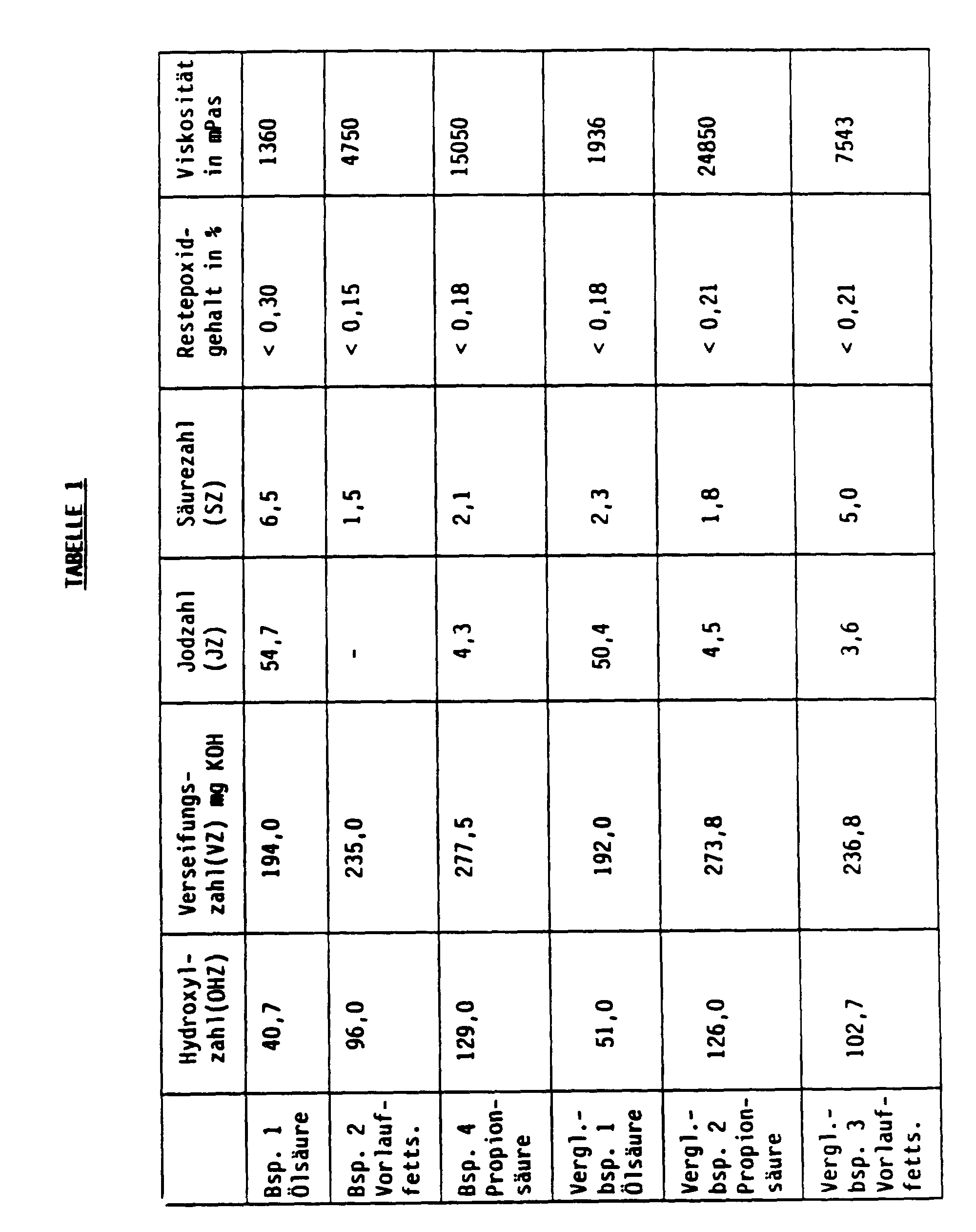

## Patentansprüche

1. Verfahren zur Herstellung von Esterpolyole enthaltenden Reaktionsmischungen durch ringöffnende Umsetzung von epoxidierten Estern und/oder Alkoholen mit Carbonsäuren, dadurch gekennzeichnet, daß man zur Herstellung vergleichsweise niedrigviskoser Umsetzungsprodukte die Carbonsäuren in wenigstens etwa stöchiometrischen Mengen zum Epoxidgehalt des/der Reaktanden vorlegt, und die epoxidierte Reaktionskomponente derart zeitverzögert eindosiert, daß substantielle Anteile nicht abreagierter Epoxidgruppen im Reaktionsgemisch vermieden werden.

2. Verfahren nach Anspruch 1, dadurch gekennzeichnet, daß die epoxidierten Ester und/oder Alkohole zu den auf Temperaturen über 80 °C und unter 300 °C, bevorzugt über 100 °C und unter 270 °C erwärmten Carbonsäuren und insbesondere unter intensivem Durchmischen des Reaktionsgemisches zeitverzögert eindosiert werden.

3. Verfahren nach einem der Ansprüche 1 und 2, dadurch gekennzeichnet, daß die epoxidierten Ester und/oder Alkohole in Mengen bis vorzugsweise 1:10, bevorzugt in höchstens etwa äquimolaren Mengen und insbesondere in geringem Unterschuß zu vorgelegten Carbonsäuren zeitverzögert eindosiert werden.

4. Verfahren nach einem der Ansprüche 1 bis 3, dadurch gekennzeichnet, daß die zeitverzögerte Zugabe der Epoxidgruppen enthaltenden Komponenten so durchgeführt wird, daß die Epoxidgehalte im Reaktionsgemisch unter 50 Gew.-% gehalten werden.

5. Verfahren nach einem der Ansprüche 1 bis 4, dadurch gekennzeichnet, daß überschüssige Carbonsäureanteile von den Esteropolyolgemischen vorzugsweise destillativ und unter Vakuum abgetrennt werden.

6. Verfahren nach einem der Ansprüche 1 bis 5, dadurch gekennzeichnet, daß zur Ringöffnung vorzugsweise einbasige Carbonsäuren vorgelegt werden.

7. Verfahren nach einem der Ansprüche 1 bis 6, dadurch gekennzeichnet, daß aromatische, aliphatische, verzweigte und/oder unverzweigte Carbonsäuren, vorzugsweise mit bis zu 40 C-Atomen, bevorzugt mit bis zu 36 C-Atomen und insbesondere mit bis zu 22 C-Atomen vorgelegt werden.

8. Verfahren nach einem der Ansprüche 1 bis 7, dadurch gekennzeichnet, daß gesättigte und/oder ungesättigte Carbonsäuren, insbesondere ungesättigte Carbonsäuren vorgelegt werden.

9. Verfahren nach einem der Ansprüche 1 bis 9, dadurch gekennzeichnet, daß epoxidierte Ester eingesetzt werden, die vorzugsweise im Mittel mehr als eine Epoxidgruppe, insbesondere 2 und mehr Epoxidgruppen im Molekül enthalten.

10. Verfahren nach einem der Ansprüche 1 bis 9, dadurch gekennzeichnet, daß epoxidierte Ester von 1- bis 4-funktionellen Alkoholen, bevorzugt von 2- und/oder 3-funktionellen Alkoholen und insbesondere Triglyceride eingesetzt werden.

11. Verfahren nach einem der Ansprüche 1 bis 10, dadurch gekennzeichnet, daß Ester von epoxidierten Carbonsäuren und/oder epoxidierten Alkoholen, vorzugsweise Ester epoxidierter Carbonsäuren und insbesondere epoxidierter Fettsäuren eingesetzt werden.

12. Verfahren nach einem der Ansprüche 1 bis 11, dadurch gekennzeichnet, daß epoxidierte Alkohole mit 3 bis 32 C-Atomen, bevorzugt mit über 12 und insbesondere bis 22 C-Atomen eingesetzt werden.

13. Verfahren nach einem der Ansprüche 1 bis 12, dadurch gekennzeichnet, daß Gemische mit Viskositäten hergestellt werden, die bis zu 50 % und bevorzugt zwischen 10 und 40 % niedriger sind als die Viskositäten der nach dem Eintopfverfahren hergestellten Gemische.

14. Verfahren nach einem der Ansprüche 1 bis 13, dadurch gekennzeichnet, daß die hergestellten Esterpolyolmischungen im statistischen Mittel mehr als eine, bevorzugt mehr als 1,5 freie Hydroxylgruppen enthalten.

15. Verfahren nach einem der Ansprüche 1 bis 14, dadurch gekennzeichnet, daß die hergestellten Esterpolyolmischungen vorzugsweise überwiegend in benachbarter Stellung zu den freien Hydroxylgruppen Estergruppen der vorgelegten Carbonsäure enthalten.

16. Mischungen von Esterpolyolen, herstellbar nach dem Verfahren der Ansprüche bis 15, dadurch gekennzeichnet, daß sie zum überwiegenden Anteil Esterpolyole enthalten, die freie Hydroxylgruppen in Nachbarstellung zur Estergruppe aufweisen.

17. Mischungen von Esterpolyolen nach Anspruch 16, dadurch gekennzeichnet, daß sie Estergruppen von Carbonsäuren von 1 bis zu 40 C-Atomen und vorzugsweise solche des Bereichs von 8 bis 22 C-Atomen enthalten.

## Claims

1. A process for the production of reaction mixtures containing ester polyols by ring-opening reactions of epoxidized esters and/or alcohols with carboxylic acids, characterized in that, to obtain comparatively low-viscosity reaction products, the carboxylic acids are initially introduced in at least substantially stoichiometric quantities to the epoxide content of the reactant(s) and the epoxidized reaction component is subsequently introduced with such a time delay that substantial numbers of unreacted epoxide groups in the reaction mixture are avoided.

2. A process as claimed in claim 1, characterized in that the epoxidized esters and/or alcohols are added with time delay to the carboxylic acids heated to temperatures above 80°C and below 300°C and preferably to temperatures above 100°C and below 270°C, more particularly with intensive stirring of the reaction mixture.

3. A process as claimed in claim 1 or 2, characterized in that the epoxidized esters and/or alcohols are added with time delay to the carboxylic acids introduced in quantities of preferably up to 1:10, preferably in at most substantially equimolar quantities and, more particularly, in slightly less than the equimolar quantity to the carboxylic acids initially introduced.

4. A process as claimed in any of claims 1 to 3, characterized in that the components containing epoxide groups are added with such a time delay that the epoxide contents in the reaction mixture are kept below 50% by weight.

5. A process as claimed in any of claims 1 to 4, characterized in that excess carboxylic acid is removed from the ester polyol mixtures, preferably by distillation and **in vacuo**.

6. A process as claimed in any of claims 1 to 5, characterized in that preferably monobasic carboxylic acids are initially introduced for the ring-opening reaction.

7. A process as claimed in any of claims 1 to 6, characterized in that aromatic, aliphatic, branched and/or unbranched carboxylic acids containing up to 40 carbon atoms, preferably up to 36 carbon atoms and, more preferably, up to 22 carbon atoms are initially introduced.

8. A process as claimed in any of claims 1 to 7, characterized in that saturated and/or unsaturated carboxylic acids, more particularly unsaturated carboxylic acids, are initially introduced.

9. A process as claimed in any of claims 1 to 9, characterized in that epoxidized esters preferably containing an average of more than one epoxide group and, more preferably, two and more epoxides groups per molecule are used.

10. A process as claimed in any of claims 1 to 9, characterized in that epoxidized esters of monohydric to tetrahydric alcohols and preferably dihydric and/or trihydric alcohols and, more particularly, triglycerides are used.

11. A process as claimed in any of claims 1 to 10, characterized in that esters of epoxidized carboxylic acids and/or epoxidized alcohols, preferably esters of epoxidized carboxylic acids and, more particularly, epoxidized fatty acids are used.

12. A process as claimed in any of claims 1 to 11, characterized in that epoxidized alcohols containing 3 to 32 carbon atoms, preferably more than 12 carbon atoms and, more preferably, up to 22 carbon atoms are used.

13. A process as claimed in any of claims 1 to 12, characterized in that mixtures having viscosities up to 50% and preferably between 10 and 40% lower than those of mixtures obtained by the one-pot method are produced.

14. A process as claimed in any of claims 1 to 13, characterized in that, on a statistical average, the ester polyol mixtures produced contain more than 1 and preferably more than 1.5 free hydroxyl groups.

15. A process as claimed in any of claims 1 to 14, characterized in that the ester polyol mixtures produced preferably contain ester groups of the carboxylic acid initially introduced predominantly adjacent the free hydroxyl groups.

16. Mixtures of ester polyols obtainable by the process claimed in claims 1 to 15, characterized in that they predominantly contain ester polyols containing free hydroxyl groups in the adjacent position to the ester group.

17. Mixtures of ester polyols as claimed in claim 16, characterized in that they contain ester groups of C₁₋₄₀ and preferably C₈₋₂₂ carboxylic acids.

## Revendications

1. Procédé de fabrication de mélanges réactionnels renfermant des esterpolyols, par réaction d'ouverture de cycle d'esters et/ou d'alcool époxydés avec des acides carboxyliques, caractérisé en ce que pour la préparation de produits de réaction de viscosité relativement basse, on dispose préalablement les acides carboxyliques en quantités au moins à peu près stoechiométriques par rapport à la teneur en époxyde du ou des réactifs et on ajoute progressivement les composants réactionnels époxydés avec un retard tel que des fractions substantielles de groupes époxy n'ayant pas réagi complètement sont évités dans le mélange réactionnel.

2. Procédé selon la revendication 1, caractérisé en ce que les esters et/ou les alcools époxydés sont ajoutés avec un certain retard aux acides carboxyliques chauffés à des températures supérieures à 80 °C et inférieures à 300°C, de préférence supérieures à 100 °C et inférieures à 270°C et, en particulier, sous agitation intense du mélange réactionnel.

3. Procédé selon une des revendications 1 ou 2, caractérisé en ce que les esters et/ou les alcools époxydés sont ajoutés progressivement avec un certain retard aux acides carboxyliques préalablement disposés, en quantités allant jusqu'à 1:10, de préférence en quantités tout au plus équimolaires et, en particulier, en faible déficit

4. Procédé selon une des revendications 1 à 3, caractérisé en ce que l'adjonction avec retard des composants contenant des groupes époxy est opérée de manière telle que la teneur en époxyde dans le mélange réactionnel est maintenue à une valeur inférieure à 50 % en poids.

5. Procédé selon une des revendications 1 à 4, caractérisé en ce que les fractions d'acide gras en excès sont séparées des mélanges d'esterpolyols, de préférence par distillation et dans le vide.

6. Procédé selon une des revendications 1 à 5, caractérisé en ce que des acides carboxyliques monobasiques sont de préférence disposés préalablement pour l'ouverture de cycle.

7. Procédé selon une des revendications 1 à 6, caractérisé en ce que des acides carboxyliques aromatiques, aliphatiques, ramifiés et/ou linéaires, comportant jusqu'ã 40 atomes de C, de préférence jusqu'à 36 atomes de C et, en particulier, jusqu'à 22 atomes de C sont disposés préalablement.

8. Procédé selon une des revendications 1 à 7, caractérisé en ce que des acides carboxyliques saturés et/ou insaturés, en particulier des acides carboxyliques insaturés, sont disposés préalablement.

9. Procédé selon une des revendications 1 à 8, caractérisé en ce que l'on met en oeuvre des esters époxydés, dont la molécule renferme de préférence en moyenne plus de 1 groupe époxy, en particulier 2 ou plus de 2 groupes époxy.

10. Procédé selon une des revendications 1 à 9, caractérisé en ce que l'on met en oeuvre des esters époxydés d'alcools mono- à tétravalents, de préférence d'alcools di-et/ou trivalents et, en particulier, des triglycérides.

11. Procédé selon une des revendications 1 à 10, caractérisé en ce que l'on met en oeuvre des esters d'acides carboxyliques et/ou d'alcools époxydés, de préférence des esters d'acides carboxyliques époxydés et, en particulier, d'acides gras époxydés.

12. Procédé selon une des revendications 1 à 11, caractérisé en ce que l'on met en oeuvre des alcools époxydés comportant 3 à 32 atomes de C, de préférence plus de 12 et, en particulier, jusqu'à 22 atomes de C.

13. Procédé selon une des revendications 1 à 12, caractérisé en ce que l'on fabrique des mélanges dont la viscosité est jusqu'à 50 %, de préférence de 10 à 40 % inférieure à celle des mélanges fabriqués selon le procédé à un récipient.

14. Procédé selon une des revendications 1 à 13, caractérisé en ce que les mélanges d'esterpolyols fabriqués renferment en moyenne statistique plus de 1, de préférence plus de 1,5 groupe hydroxyle libre.

15. Procédé selon une des revendications 1 à 14, caractérisé en ce que les mélanges d'esterpolyols fabriqués renferment des groupes ester de l'acide carboxylique disposé préalablement, de préférence en majeure partie en position voisine des groupes hydroxyle libres.

16. Mélanges d'esterpolyols confectionnables selon le procédé des revendications 1 à 15, caractérisés en ce qu'ils renferment en majeure partie des esterpolyols comportant des groupes hydroxyle libre en position voisine par rapport au groupe ester.

17. Mélanges d'esterpolyols selon la revendication 16, caractérisés en ce qu'ils renferment des groupes ester d'acides carboxyliques de 1 à 40 atomes de C, et de préférence de ceux situés dans la plage s'étendant de 8 à 22 atomes de C.
